# EUROPEAN PATENT APPLICATION

(11) **EP 4 778 470 A1**
(43) Date of publication of application: **22.07.2026**
(21) Application number: 25152368.4
(22) Date of filing: 16.01.2025
(51) Int. Cl.: A61B 8/00, A61B 8/08

(54) **ADHERABLE ULTRASOUND PROBE**

(71) Applicant: Nederlandse Organisatie voor toegepast- natuurwetenschappelijk onderzoek TNO, 2595 DA 's-Gravenhage (NL)
(72) Inventor: BAKKER, Maarten Herman, 2595 DA 's-Gravenhage (NL); KIRCHNER, Gerwin, 2595 DA 's-Gravenhage (NL); SOUNDARARAJAN, Anand, 2595 DA 's-Gravenhage (NL); OSTENDORF, Pim Reinier, 2595 DA 's-Gravenhage (NL); KAWASAKI, Shinnosuke, 2595 DA 's-Gravenhage (NL)
(74) Representative: V.O.

(57) **Abstract**

An ultrasound probe (100) for measuring internal structures (I) under a skin surface (A). The ultrasound probe (100) comprises an acoustic interface (10a), an ultrasound transducer (10), an adhesive layer (11), and a temporary liner (12). The adhesive layer (11) is configured to adhere the ultrasound transducer (10) to the skin surface (A) for measuring the internal structures (I) under the skin surface (A) from a fixated position (Af) on the skin surface (A). The temporary liner (12) is removed from the adhesive layer (11) to expose the adhesive layer (11) for said adhering of the ultrasound transducer (10) to the skin surface (A). The temporary liner (12) is formed of an acoustically transmissive material enabling the ultrasound transducer (10) to measure the internal structures (I) under the skin surface (A) from a variable position (Av) while the temporary liner (12) is covering the adhesive layer (11).

## Description

### TECHNICAL FIELD AND BACKGROUND

The present disclosure relates to ultrasound probes, ultrasound systems, and methods of using such probes and systems.

Ultrasound imaging is widely used in medicine for its non-invasive nature, real-time visualization capabilities, and versatility across various medical fields. However, its effectiveness often depends on the accurate and consistent positioning. Proper placement may be important for obtaining reliable imaging or measurement data of anatomical structures beneath the tissue surface.

In general, the position of an ultrasound probe may be maintained by temporary attachment means such as belts, strings, bandages; or more permanent attachment means such as adhesives. However, these solutions present certain challenges. On the one hand, belts, strings, and bandages may require wrapping around the patient's body, which can be uncomfortable and time-consuming to apply. On the other hand, adhesives may be used to easily fixate an ultrasound probe to the skin, e.g. in the form of a patch. However, it may be challenging to find the correct position for the ultrasound probe, and once applied to the skin, the ultrasound probe adhered to the skin cannot be easily repositioned.

There remains a need for alleviating problems associated with known ultrasound devices and methods while maintaining at least some of their advantages. For example, there is a need for improving secure and reliable positioning of an ultrasound probe while maintaining patient comfort, ease of application, consistent imaging or measurement quality, and enhanced repositioning flexibility to improve the usability and adaptability of ultrasound technology.

### SUMMARY

Aspects of the present disclosure relate to an ultrasound probe for measuring internal structures under a skin surface. The ultrasound probe comprises an acoustic interface, an ultrasound transducer, an adhesive layer, and a temporary liner. The ultrasound transducer is configured to generate ultrasound signals, and transmit the generated ultrasound signals, via the acoustic interface, into the skin surface, and/or receive, via the acoustic interface, ultrasound signals that have interacted with the internal structures, and measure the received ultrasound signals. The adhesive layer is configured to adhere the ultrasound transducer via the acoustic interface to the skin surface for measuring the internal structures under the skin surface from a fixated position on the skin surface. The temporary liner is at least initially covering the adhesive layer. In one aspect of the present disclosure, the temporary liner is configured to be removed from the adhesive layer to expose the adhesive layer for said adhering of the ultrasound transducer to the skin surface. The temporary liner is formed of an acoustically transmissive material, having acoustic wave transmissibility configured to allow passage of the ultrasound signals, generated by the ultrasound transducer, into the skin surface, and/or passage of the ultrasound signals, received from the skin surface, after interacting with the internal structures, to the ultrasound transducer, thereby enabling the ultrasound transducer to measure the internal structures under the skin surface from a variable position on the skin surface while the temporary liner is covering the adhesive layer.

By providing an ultrasound probe with a temporary liner formed of an acoustically transparent material, the ultrasound probe can be easily repositioned over a skin surface while maintaining acoustic contact to the skin surface for measuring internal structures underneath. Advantageously, by peeling off the temporary liner and thereby exposing an adhesive layer, the position of the ultrasound probe can be fixated on the skin surface for continuous and/or long-term measuring of internal structures under the skin surface.

By providing the temporary liner with a wetted and/or oily interface, its slidability over the skin surface can be improved, e.g. by reducing the shear strength required to slide the ultrasound probe over the skin surface. Furthermore, the acoustic contact between the acoustic interface of the ultrasound probe and the skin surface may also be improved, e.g. by filling in air gaps between the acoustic interface and the skin surface.

By providing the temporary liner with a soft conformable material, the acoustic contact of the acoustic interface to the skin surface can be further improved, e.g. by pressing out air bubbles between the acoustic interface and the skin surface. By attaching the conformable material to a carrier layer, the mechanical strength of the conformable material on the side opposite to the skin surface can be enhanced.

By forming the conformable material of a hydrogel and/or organogel, acoustic impedance and shape of the ultrasound transducer can be matched to the skin surface. The hydrogel may be wetted and the organogel oiled to increase slidability of the conformable material over the skin surface.

By providing the temporary liner with a retention layer, the shear strength between the conformable material and the carrier layer can be increased to prevent dislocation of the conformable material from the carrier layer. By providing a mesh structure to the retention layer, bonding strength of the conformable material to the carrier layer can be increased. By arranging the retention layer in segments, dislocation of the conformable material from the retention layer can be prevented during repositioning of the ultrasound probe.

By forming the adhesive layer of an absorbing material, acoustic contact between the ultrasound probe and the skin surface can be improved after removing the temporary liner, e.g. by filling in air gaps with liquid residues from the conformable material and/or the skin surface. By forming the adhesive layer of a relatively strong adhesive, the ultrasound probe can remain self-supported on the skin surface for continuous and/or intermittent measurement of internal structures without the need for additional fixtures.

By providing the temporary liner with a lip, the contact area where the peeling force is applied and/or the angle of the peeling force can be increased to ease at least the initiation of peeling off the liner from the adhesive layer. By dividing the temporary liner into smaller, independent sections, the forces required to peel off the temporary liner from the adhesive layer are reduced and the peeling process is better controlled.

Advantageously, by integrating the adhesive layer into the temporary liner, the temporary liner can be easily applied *in situ* to existing ultrasound probes without the need of their structural modifications. By integrating the ultrasound probe in a patch, correct position can be easily found and secured for long-term imaging and/or measuring of internal structures from a fixated position on the skin surface.

By integrating the ultrasound probe into an ultrasound system, a controller and/or signal processor can be adapted to take acoustic effects of the temporary liner into the account, also the presence or absence of the liner can be automatically recognized by the ultrasound system.

Advantageously, the ultrasound probe and/or the ultrasound system with the slidable temporary liner formed of an acoustically transmissive material is used in a method of localizing and subsequently imaging and/or measuring internal structures under a skin surface. By continuously and/or intermittently imaging and/or measuring internal structures below the skin surface while moving, e.g. sliding, the ultrasound probe over the skin surface, the internal structure of interest can be found, and subsequently the position to image and/or measure the found internal structure can be fixated.

### BRIEF DESCRIPTION OF DRAWINGS

These and other features, aspects, and advantages of the apparatus, systems and methods of the present disclosure will become better understood from the following description, appended claims, and accompanying drawing wherein:
FIGs 1A-1C illustrate an adherable ultrasound probe;
FIGs 2A-2C illustrate a temporary liner;
FIGs 3A-3E illustrate removal of the temporary liner;
FIGs 4A-4C illustrate a patch with the temporary liner.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

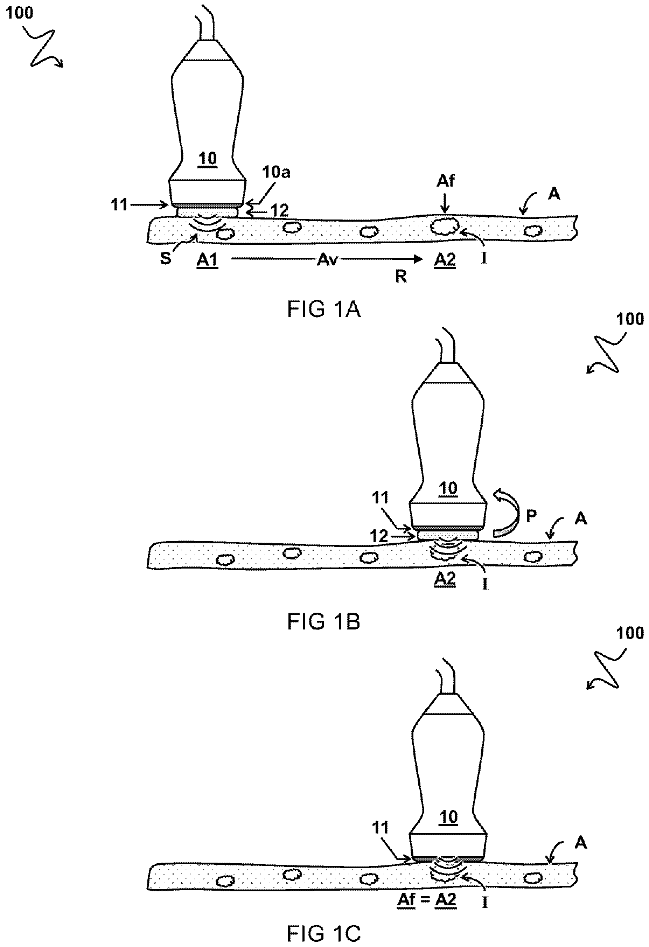

FIGs 1A-1C illustrate an adherable ultrasound probe 100 for measuring internal structures I under a skin surface A. Typically, the internal structures I comprise anatomical parts of an animal or a human body, e.g. organs, blood vessels, nerves, bones, fetus, physiological effects, or pathological tissues, e.g. tumors, atherosclerotic plaques, etc. Alternatively, or in addition, the ultrasound probe 100 can be used to monitor physiological effects, e.g. heartbeat, blood flow, breathing, etc.

The ultrasound probe 100 comprises an ultrasound transducer 10. Typically, the ultrasound transducer 10 has an acoustic interface 10a. In one embodiment, the ultrasound transducer 10 is configured to generate ultrasound signals S, and transmit the generated ultrasound signals, via the acoustic interface 10a, into the skin surface A. In another or further embodiment, the ultrasound transducer 10 is configured to receive, via the acoustic interface 10a, ultrasound signals S that have interacted with the internal structures I, and measure the received ultrasound signals S. Preferably, the ultrasound signals S comprise ultrasound waves having an acoustic frequency in the range from one up to forty megahertz, more preferably in a range from five up to twelve megahertz. Preferably, the ultrasound signals S are transmitted and received by the same ultrasound transducer 10. Alternatively, or in addition, a plurality of ultrasound transducers is used for transmitting and receiving the ultrasound signals S, or the ultrasound signals are generated by alternative means, e.g. photoacoustic effect, and the ultrasound transducer 10 is configured only to receive ultrasound signals S. In one embodiment, the ultrasound transducer 10 comprises an array of ultrasound elements for electronic scanning and/or beamforming of the ultrasound signals S from the internal structures I.

As described herein, the adhesive layer 11 is configured to adhere the ultrasound transducer 10 via the acoustic interface 10a to the skin surface A for measuring the internal structures I under the skin surface A from a fixated position Af on the skin surface A. Accordingly, the ultrasound probe 100 comprises a temporary liner 12 at least initially covering the adhesive layer 11. The temporary liner 12 is configured to be removed from the adhesive layer 11 to expose the adhesive layer 11 for said adhering of the ultrasound transducer 10 to the skin surface A. Preferably, the adhesive layer 11 passes through more than forty, more preferably sixty, and most preferably eighty or more percent of acoustic energy comprised in the ultrasound signals S. Accordingly, the thickness of the adhesive layer 11 is in a range from ten micrometers up to one hundred micrometers or more. Preferably, the adhesive strength of the adhesive layer 11 to the skin surface A is at least a factor of two, more preferably five, and most preferably ten times larger than the adhesive strength of the temporary liner 12. For example, the adhesive layer 11 may comprise Duplomed 22507, MED 6513SI, adhesive hydrogel, acrylic bioadhesive, or other kinds of skin-friendly adhesive.

In some embodiments, the temporary liner 12 is formed of an acoustically transmissive material. In one embodiment, the temporary liner 12 is formed of an acoustically transmissive material, having acoustic wave transmissibility configured to allow passage of the ultrasound signals S, generated by the ultrasound transducer 10, into the skin surface A. In another or further embodiment, the temporary liner 12 is formed of an acoustically transmissive material, having acoustic wave transmissibility configured to allow passage of the ultrasound signals S, received from the skin surface A, after interacting with the internal structures I, to the ultrasound transducer 10. Preferably, the temporary liner 12 passes through more than ten, more preferably thirty, and most preferably eighty or more percent of acoustic energy comprised in the ultrasound signals S. Accordingly, the thickness of the temporary liner 12 is in a range from fifteen micrometers up to ten millimeters or more. Preferably, the temporary liner 12 comprises siliconized polyester (LDPE silicone). Alternatively, or in addition, other material preventing sticking on at least the side of the skin surface A can be used.

Advantageously, the ultrasound transducer 10 can measure the internal structures I under the skin surface A from a variable position Av on the skin surface A while the temporary liner 12 is covering the adhesive layer 11. As it will be understood, the variable position Av is non-fixated on the skin surface. The fixated position Af is a position on the skin surface providing fixed field of view on internal structures I underneath the skin surface A. Alternatively, or in addition, the fixated position Af may be an at least partially fixated position, e.g. exclusively restricted to rotation and/or tilt of the ultrasound probe 100.

In some embodiments, the temporary liner 12 is separable from the adhesive layer 11 by a relatively weak adhesive force per surface area as compared to a relatively strong adhesive force between the adhesive layer 11 and the acoustic interface 10a of the ultrasound transducer 10, e.g. lower in Newton per square meter by at least a factor of two, three, five, ten or more. Other types of separation may also be contemplated. Preferably, the aforementioned relatively weak adhesive force is less than twenty Newton per centimeter, preferably less than fifteen Newton per square centimeter, more preferably less than ten N/cm, and most preferably less than five N/cm. In another or further embodiment, the aforementioned relatively strong adhesive force between the adhesive layer 11 and the acoustic interface 10a, is more than five Newton per centimeter, preferably more than 10 N/cm, or even ≥twenty N/cm. In general, the adhesive strength between layers can be measured in various ways. For example, the aforementioned values of adhesive strength can be measured using a 90° peel test peel test at 100 mm/min as described by the ISO 8510-1:2022 standard test temperature 23 ± 2 °C and relative humidity 50 ± 5%.

In some embodiments, the temporary liner 12 is a slidable temporary liner 12 configured to reposition R, by sliding, the ultrasound probe 100 on the skin surface A while maintaining acoustic contact of the ultrasound transducer 10 with the skin surface A. For example, as shown in FIGs 1A-1B, the ultrasound probe 100 may be repositioned from a first position A1 on the skin surface A to a second position A2 on the skin surface A, while continuously measuring internal structures I at different locations below the skin surface A between the first position A1 and the second position A2. For example, as shown in FIG 1C, the ultrasound probe 100 may be fixated at the second position A2 (= Af) to continue measuring internal structures I below the skin surface A at the fixated position Af. Alternatively, or in addition, the slidable temporary liner 12 is configured to reposition R the ultrasound probe 100 by translation or rotation over the skin surface A. In one embodiment, the temporary liner 12 can slide over a distance of one up to ten, twenty or more centimeters over the skin surface A. Advantageously, slidability of the temporary liner 12 over the skin surface A can be enhanced by liquid and/or gel applied on the temporary liner 12, e.g. water or oil. The slidability of the temporary liner 12 can be measured as an ability to resist forces that cause sliding or deformation along a plane parallel to the applied force, e.g. shear strength expressed in Newton per centimeter squared. In one embodiment, the shear strength of the slidable temporary liner 12 is less than one newton per squared centimeter, preferably less than half a newton per squared centimeter to ensure no damage to the skin surface A, or even less in the case of a sensitive skin. In another or further embodiment, the shear strength between the temporary liner 12 and the adhesive layer 11 is substantially higher than the shear strength between the temporary liner 12 and the skin surface A, e.g. a factor of two, ten, up to twenty of more higher.

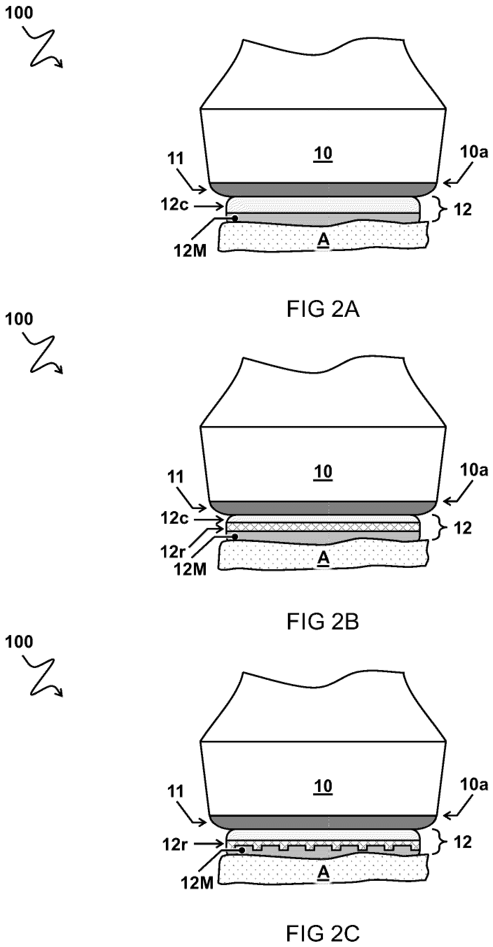

FIGs 2A-2C illustrate a temporary liner 12 comprising a carrier layer 12c arranged in contact with the adhesive layer 11 and a conformable material 12M covering the carrier layer 12c. Advantageously, e.g. as shown in FIG 2A, the conformable material 12M is configured to conform to the skin surface A to improve acoustic contact between the ultrasound probe 100 and the skin surface, e.g. by filling in air gaps and/or pressing out air bubbles between the acoustic interface 10a and the skin surface A. Accordingly, the thickness of the conformable material 12M is in a range from one millimeter up to ten millimeters or more. Preferably, the conformable material 12M comprises (solid) hydrogel or organogel. Alternatively, or in addition, another kind of soft solid material having acoustic properties (e.g. speed of sound) close to that of water can be used. In one embodiment, the carrier layer 12c is configured to provide mechanical support to the conformable material 12M during repositioning R of the ultrasound transducer 10 over the skin surface A. In another or further embodiment, e.g. as shown in FIG 2B, the temporary liner 12 comprises a retention layer 12r fused to the carrier layer 12c and configured to retain the position of the conformable material 12M with respect to the carrier layer 12c during repositioning R of the ultrasound transducer 10. Advantageously, the retention layer 12r is configured to increase the shear strength between the carrier layer 12c and the conformable material 12M to prevent the conformable material 12M from sliding off the carrier layer 12c. In one embodiment, e.g. , as shown in FIG 2C, the retention layer 12r comprises a mesh structure configured to incorporate a portion of the conformable material 12M for bonding the conformable material 12M to the carrier layer 12c during the repositioning R. Alternatively, or in addition, chemical bonding can be used to attach the conformable material 12M to the carrier layer 12c. In some embodiments, the retention layer 12r is arranged in segments comprising the conformable material 12M to retain the position of the conformable material 12M in the sliding direction with respect to the carrier layer 12c during the repositioning R.

Advantageously, the adhesive layer 11 is configured to absorb liquid material, e.g. water or oil, after removing the temporary liner 12 from the adhesive layer 11, thereby promoting adhesion of the adhesive layer 12 to the skin surface A and maintaining acoustic contact between the ultrasound transducer 10 and the skin surface A. In one embodiment, the adhesive layer 11 absorbs residues of hydrogel of organogel from the skin surface A to improve adhesion and/or maintain acoustic contact between the acoustic interface 10a and the skin surface A. Alternatively, or in addition, the adhesive layer 11 absorbs water or oil from the skin surface A. In some embodiments, the adhesive strength of the adhesive layer 11 is configured to self-support the ultrasound probe 100 on the skin surface A for continuous and/or intermittent measurement of internal structures I from the fixed position Af. Consequently, there is no need for additional fixtures as the ultrasound probe 100 is "free-standing.

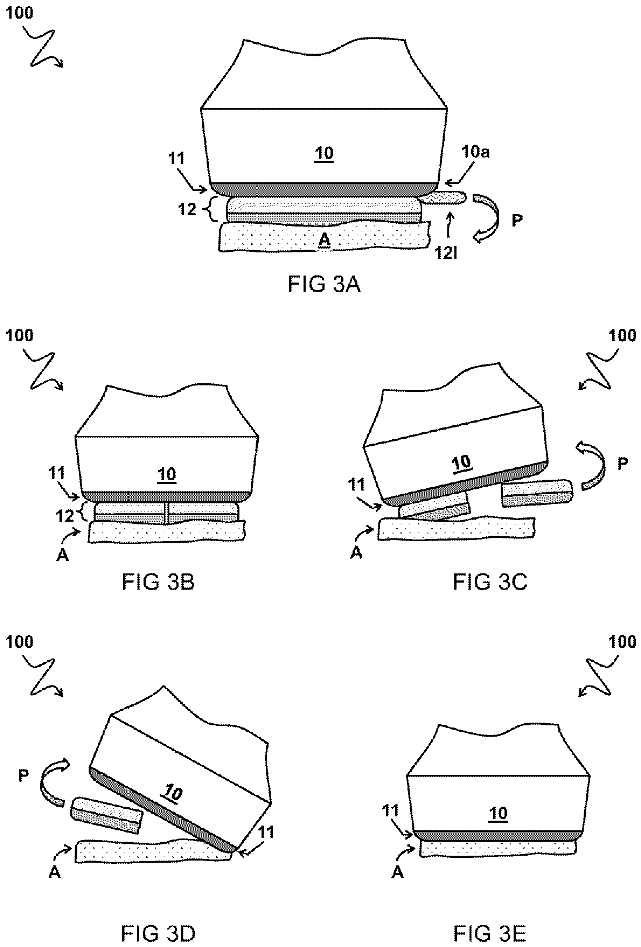

FIGs 3A-3E illustrate removal of the temporary liner 12. In some embodiments, e.g. as shown in FIG 3A, the temporary liner 12 comprises a lip 121 configured to facilitate peeling off P the temporary liner 12 from the adhesive layer 11 while acoustic contact of the ultrasound transducer 10 with the skin surface A is at least partially maintained. Accordingly, the geometry of the lip 121 is configured to increase shear stress during peeling off P the temporary liner 12 by reducing the contact area where the peeling force is applied and/or altering the angle of the peeling force. In other or further embodiments, e.g. as shown in FIG 3B, the temporary liner 12 is divided into sections for sequentially peeling off P the respective sections of the temporary liner 12 without repositioning the ultrasound probe 100 from the fixated position Af. For example, the first section is removed from a first side after the ultrasound probe 100 is tilted and at least partially pushed by a second side into the skin surface A, e.g. as illustrated in FIG 3C. Subsequently, the second section is removed from the second side after tilting and at least partially pushing the ultrasound probe 100 by the first side into the skin surface A, e.g. as illustrated in FIG 3D, to adhere the ultrasound probe 100 to the skin surface A, e.g. as illustrated in FIG 3E.

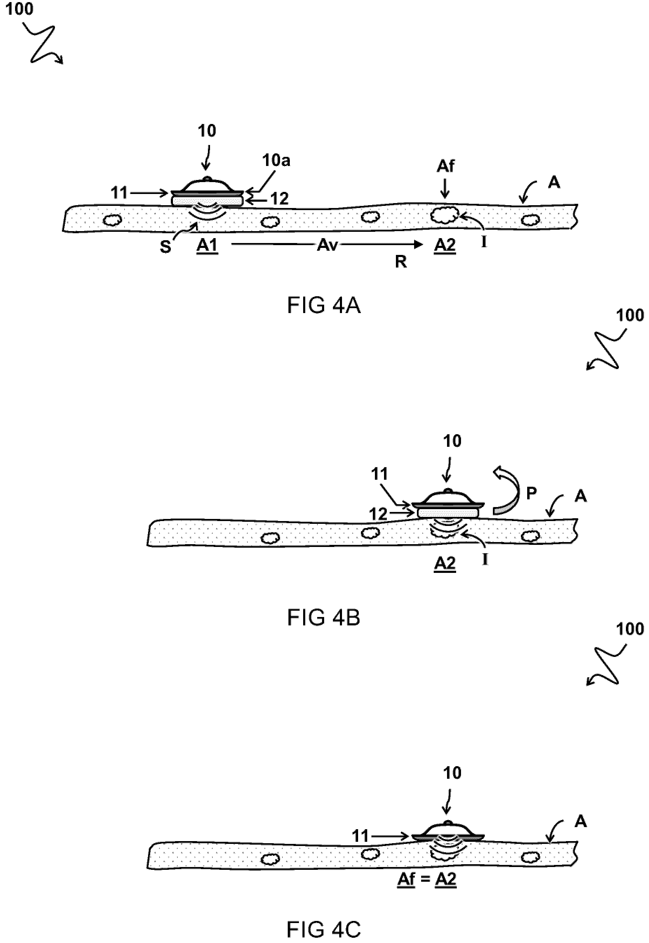

In one embodiment, the adhesive layer 11 is integrated into the temporary liner 12 and configured to adhere the temporary liner 12 to the ultrasound transducer 10 during repositioning R of the ultrasound transducer 10 over the skin surface A from the first position A1 to the second position A2. As described herein, the adhesive layer 11 is also configured to adhere the ultrasound transducer 10 to the skin surface A at the second position A2 for measuring the internal structures I under the skin surface A from the fixated position Af. Advantageously, by integrating the adhesive layer into the temporary liner, the liner can be easily applied in situ to an existing ultrasound probe 100. Alternatively, or in addition, the temporary liner 12 is adhered to the ultrasound probe 100 by an additional layer of adhesive applied to the ultrasound probe 100. FIGs 4A-4C illustrate a patch with the temporary liner. In some embodiments, the ultrasound probe 100 is comprised in a patch for long-term measuring of the internal structures I from the fixated position Af. As described herein, the patch is configured for long-term monitoring of anatomical parts of an animal or human body.

In some embodiments, the ultrasound probe 100 is comprised in an ultrasound system for measuring internal structures I under a skin surface A. Accordingly, the ultrasound system comprises the ultrasound probe 100 with the temporary liner 12. Typically, the ultrasound probe 100 comprises an electrical signal generator configured to generate electrical signals to drive the ultrasound transducer 10 to generate the ultrasound signals S. Typically, the ultrasound system also comprises a measurement circuit configured to measure electrical signals received from the ultrasound transducer 10. The ultrasound system may comprise a controller and/or signal processor configured to control the electrical signal generator and/or process the ultrasound signals measured by the measurement circuit. Accordingly, one or more of the controller, electrical signal generator, measurement circuit, and signal processor are configured and/or adapted to enable the ultrasound system to measure, using the ultrasound probe 100, also through the temporary liner 12, the internal structures I under the skin surface A.

The ultrasound probe 100 and/or ultrasound system described herein may be used in an ultrasound imaging and/or measuring method. Accordingly, the method comprises providing the ultrasound probe 100 with the temporary liner 12 covering the adhesive layer 11, contacting a skin surface A at a first position A1 by the ultrasound probe 100 with the temporary liner 12 disposed between the adhesive layer 11 and the skin surface A, and measuring internal structures I under the skin surface A at the first position A1 using ultrasound signals S transmitted through the temporary liner 12 between the acoustic interface 10a and the skin surface A. The ultrasound probe 100 may be repositioned from the first position A1 to a second position A2 on the skin surface A, different from the first position A1, with the temporary liner 12 still disposed between the adhesive layer 11 and the skin surface A. The internal structures I under the skin surface A can be measured at the second position A2 using ultrasound signals S transmitted through the temporary liner 12 between the acoustic interface 10a and the skin surface A. Advantageously, the temporary liner 12 can be removed and the ultrasound probe 100 can be adhered by means of the adhesive layer 11 to the skin surface A at the second position A2. Consequently, the ultrasound probe 100 adhered to the skin surface A at the second position A2 can be used to continue measuring the internal structures I under the skin surface A using ultrasound signals S without the temporary liner 12 between the acoustic interface 10a and the skin surface A. In one embodiment, the ultrasound probe 100 is used to continuously or intermittently measure internal structures I below the skin surface A while the ultrasound probe 100 is moved, e.g. slid, over the skin surface A between the first position A1 and the second position A2. So, it will be understood that one or more acoustic measurements may be performed at intermittent positions between the first position A1 and second position A2. For example, a user may vary the position of the ultrasound probe 100 while performing acoustic measurements through the temporary liner 12, and remove the temporary liner 12 when a desired position on the skin surface A is located, where the ultrasound probe 100 may be arranged for continued, e.g. long-term, measurements.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise.

## Claims

1. An ultrasound probe (100) for measuring internal structures (I) under a skin surface (A), the ultrasound probe (100) comprising
an acoustic interface (10a);
an ultrasound transducer (10) configured to
generate ultrasound signals (S), and transmit the generated ultrasound signals, via the acoustic interface (10a), into the skin surface (A), and/or
receive, via the acoustic interface (10a), ultrasound signals (S) that have interacted with the internal structures (I), and measure the received ultrasound signals (S);
an adhesive layer (11) configured to adhere the ultrasound transducer (10) via the acoustic interface (10a) to the skin surface (A) for measuring the internal structures (I) under the skin surface (A) from a fixated position (Af) on the skin surface (A); and
a temporary liner (12) at least initially covering the adhesive layer (11), wherein the temporary liner (12) is configured to be removed from the adhesive layer (11) to expose the adhesive layer (11) for said adhering of the ultrasound transducer (10) to the skin surface (A);
wherein the temporary liner (12) is formed of an acoustically transmissive material, having acoustic wave transmissibility configured to allow
passage of the ultrasound signals (S), generated by the ultrasound transducer (10), into the skin surface (A), and/or
passage of the ultrasound signals (S), received from the skin surface (A), after interacting with the internal structures (I), to the ultrasound transducer (10),
wherein the acoustically transmissive material of the temporary liner (12) enables the ultrasound transducer (10) to measure the internal structures (I) under the skin surface (A) from a variable position (Av) on the skin surface (A) while the temporary liner (12) is still covering the adhesive layer (11).

2. The ultrasound probe (100) according to the preceding claim, wherein the temporary liner (12) is a slidable temporary liner (12) configured to enable repositioning (R), by sliding, the ultrasound probe (100) on the skin surface (A) while maintaining acoustic contact of the ultrasound transducer (10) with the skin surface (A).

3. The ultrasound probe (100) according to any of the preceding claims, wherein the temporary liner (12) comprises
a carrier layer (12c) arranged in contact with the adhesive layer (11); and
a conformable material (12M) covering the carrier layer (12c) and configured to conform to the skin surface (A);
wherein the carrier layer (12c) is configured to provide mechanical support to the conformable material (12M) during repositioning (R) of the ultrasound transducer (10) over the skin surface (A).

4. The ultrasound probe (100) according to the preceding claim, wherein the conformable material (12M) comprises hydrogel or organogel.

5. The ultrasound probe (100) according to the preceding claim, wherein the hydrogel or organogel comprises a liquid material, wherein the adhesive layer (11) is configured to absorb at least a portion of the liquid material after removing the temporary liner (12) from the adhesive layer (11), to promote adhesion of the adhesive layer (11) on the skin surface (A) in the presence of any of said liquid material remaining on the skin surface (A).

6. The ultrasound probe (100) according to the preceding claim, wherein the temporary liner (12) comprises a retention layer (12r) fused to the carrier layer (12c) and configured to retain the conformable material (12M) in the retention layer (12r) during repositioning (R) of the ultrasound transducer (10).

7. The ultrasound probe (100) according to the preceding claim, wherein the retention layer (12r) comprises a mesh structure configured to incorporate a portion of the conformable material (12M) for bonding the conformable material (12M) to the carrier layer (12c) during the repositioning (R).

8. The ultrasound probe (100) according to any of the two preceding claims, wherein the retention layer (12r) is arranged in segments comprising the conformable material (12M) for retaining a position of the conformable material (12M) in the sliding direction with respect to the carrier layer (12c) during the repositioning (R).

9. The ultrasound probe (100) according to any of the preceding claims, wherein an adhesive strength of the adhesive layer (11) is configured to self-support the ultrasound probe (100) on the skin surface (A) for continuous and/or intermittent measurement of internal structures (I) from the fixed position (Af).

10. The ultrasound probe (100) according to any of the preceding claims, wherein the temporary liner (12) comprises a lip (121) configured to facilitate peeling off (P) the temporary liner (12) from the adhesive layer (11) while acoustic contact of the ultrasound transducer (10) with the skin surface (A) is at least partially maintained.

11. The ultrasound probe (100) according to any of the preceding claims, wherein the temporary liner (12) is divided into sections for sequentially peeling off (P) the respective sections of the temporary liner (12) without repositioning the ultrasound probe (100) from the fixated position (Af).

12. The ultrasound probe (100) according to any of the preceding claims, wherein the ultrasound probe (100) is comprised in a patch for unsupervised continuous measuring of the internal structures (I) from the fixated position (Af).

13. An ultrasound system for measuring internal structures (I) under a skin surface (A), the ultrasound system comprising
the ultrasound probe (100) according to any of the preceding claims;
an electrical signal generator configured to generate electrical signals to drive the ultrasound transducer (10) to generate the ultrasound signals (S);
a measurement circuit configured to measure electrical signals received from the ultrasound transducer (10);
a controller and/or signal processor configured to control the electrical signal generator and/or process the ultrasound signals measured by the measurement circuit;
wherein one or more of the controller, electrical signal generator, measurement circuit, and signal processor are configured and/or adapted to enable the ultrasound system to measure, using the ultrasound probe (100), also through the temporary liner (12), the internal structures (I) under the skin surface (A).

14. A method of using an ultrasound probe (100) and/or ultrasound system according to any of the preceding claims, the method comprising
providing the ultrasound probe (100) with the temporary liner (12) covering the adhesive layer (11);
contacting a skin surface (A) at a first position (A1) by the ultrasound probe (100) with the temporary liner (12) disposed between the adhesive layer (11) and the skin surface (A);
measuring internal structures (I) under the skin surface (A) at the first position (A1) using ultrasound signals (S) transmitted through the temporary liner (12) between the acoustic interface (10a) and the skin surface (A);
repositioning (R) the ultrasound probe (100) from the first position (A1) to a second position (A2) on the skin surface (A), different from the first position (A1), with the temporary liner (12) still disposed between the adhesive layer (11) and the skin surface (A);
measuring internal structures (I) under the skin surface (A) at the second position (A2) using ultrasound signals (S) transmitted through the temporary liner (12) between the acoustic interface (10a) and the skin surface (A);
removing the temporary liner (12) and adhering the ultrasound probe (100) by means of the adhesive layer (11) to the skin surface (A) at the second position (A2); and
measuring, with the ultrasound probe (100) adhered to the skin surface (A) at the second position (A2), internal structures (I) under the skin surface (A) using ultrasound signals (S) without the temporary liner (12) between the acoustic interface (10a) and the skin surface (A).

15. The method according to the preceding claim, wherein the ultrasound probe (100) is used to measure internal structures (I) below the skin surface (A) while the ultrasound probe (100) is moved, e.g. slid, over the skin surface (A) between the first position (A1) and the second position (A2).
